# EUROPEAN PATENT SPECIFICATION

(11) **EP 1 891 205 B1**
(45) Date of publication and mention of the grant of the patent: **27.12.2017**
(21) Application number: 06772571.3
(22) Date of filing: 08.06.2006
(51) Int. Cl.: C12N 5/00, C12N 5/02, C12M 1/24

(54) **SUSPENSION CULTURE VESSELS**
SUSPENSIONSKULTURGEFÄSSE
FLACON POUR CULTURE EN SUSPENSION

(30) Priority: 15.06.2005 US 690587 P
(43) Date of publication of application: 27.02.2008
(73) Proprietor: ZHEJIANG JINYISHENGSHI BIOENGINEERING CO., LTD., Huzhou City Zhejiang 313000 (CN)
(72) Inventor: HUI, Mizhou, Thousand Oaks, CA 91360 (US)
(74) Representative: Chajmowicz, Marion
(86) International application number: PCT/US2006/022312
(87) International publication number: WO 2006/138143

(56) References cited:
- EP-A2- 0 752 470
- WO-A1-98/27195
- US-A- 4 665 035
- US-B1- 6 391 638
- US-B1- 6 991 933
- DATABASE WPI Week 197914 Thomson Scientific, London, GB; AN 1979-27030B XP002672046, -& JP 54 026972 A (TOKYO RIKAKIKAI KK) 28 February 1979 (1979-02-28)
- 'HERPES (Free Acid). Product Information' MEDIATECH, INC., [Online] XP008134817 Retrieved from the Internet: <URL:http://www.CELLGRO.COM>

## Description

### FIELD OF THE INVENTION

The present invention relates to a wide-body mammalian cell culture vessel with an inversed frusto-conical or inverted frustum bottom for reduced seed volume and better mixing with less hydro-mechanical stress and greater aeration.

### BACKGROUND OF THE INVENTION

High-density suspension culture of mammalian cells is a useful tool for protein drug or vaccine development. It often requires small-volume cell culture vessels for production of animal testing materials, and large-volume cell culture vessels for production of clinical trial material. Most cell biologists prefer simplified small volume suspension culture equipments, while scale-up professionals enjoy "easy to optimize" large-volume cell culture systems.

Tall cylinder is a typical shape for current bioreactor culture vessels and spinner bottles. Such a vessel or bottle has a height: diameter ratio of larger than 1/1 of at work volume. The surface area of the culture medium in the vessel is not enough for effective 02 uptake. Air or 02 sparging has been used to address this problem. However, overdose of air sparging often causes cell damage by foaming and bubble burst. Also, overdose of pure 02 sparging is toxic to cells. Thus, a sophisticated control tower and related dissolved oxygen (DO) probes are required to monitor and control air or pure 02 sparging. Nonetheless, it is tedious and costly to optimize the control tower or probe.

US 4 665 035 relates to shake flask aerobic fermentation apparatus and systems having baffling and closuring for microbial growths.

EP 0 752 470 relates to a method for the culturing for the culturing of ciliates in spinner flasks.

JP 5 26 972 relates to a pipe connecting device in which a pipe end part with enlarged diameter can be externally inserted to an inserting tube part of a pipe joint.

WO98/27195 relates to a receptacle for cell culture used to culture an antitumour cell transplant for gene and cell therapy.

Thus, there is a need for small volume culture vessels, particularly disposable vessels, without control tower and related DO probe, and for "easy to optimize" large volume culture vessels.

This design of this invention allows one to culture large volume of cells without using sophisticated control tower and related probes, Also, disposable or autoclavable small-volume shaker-based suspension culture systems have been developed. In addition, a prototype for an "easy to optimize" impellor-based large volume industrial inverted frusto-conical bioreactor system has also been studied.

### SUMMARY OF INVENTION

This invention features a wide-body culture vessel with an inverted frusto-conical bottom. Comparing with traditional flat-bottom bioreactor vessels, the vessel of this invention has a significant larger culture medium surface area for 02 uptake and CO2 stripping. The inverted frusto-conical bottom provides significant seed volume reduction for initiation of a cell culture process. The inverted frusto-conical bottom makes a low-positioned air sparging possible for extended air bubble traveling time course. Meanwhile, orbital shaking can be used so that the culture medium climb up onto the wall of the vessel easily with no share-force and less hydro-mechanical stress. This creates a broad thin medium layer for extended surface and greater aeration and better mixing. By using this design, plastic "single use" or glass autoclavable small-volume shaker-based suspension culture systems with no sophisticated control tower and related probes have been developed. In addition, a prototype for an "easy to optimize" impellor-based large volume industrial bioreactor system has been studied.

### BRIEF DESCRIPTION OF DRAWINGS

FIG. 1a is a drawing of 3-liter wide-body vessel with an inverted frusto-conical bottom; work volume=3 L; culture medium surface area/culture medium volume >0.143 cm2/cm<3>; minimum seeding volume=150 ml, 1/20 of marked 3-liter work volume.
Figure 1b is a photograph of a 3-liter wide-body shaker vessel with an inverted frusto-conical bottom and a traditional flat-bottom Applikon bioreactor vessel on the background.
Figure 2 is a photograph of a 150 ml wide-body shaker bottle with an inverted frusto-conical bottom; work volume = 150ml culture medium; total free air space=500ml; minimum seeding volume = 7.5ml, 1/20 of the marked work volume. A shaker platform, a flow meter, a low-positioned air bubbling device and an air pump are required to support this system. This system is mainly designed for screening production cell line candidates for their robustness and productivity, as well as seed train support.
Figure 3 is a series of drawings of wide-body suspension culture vessels with inverted frusto-conical bottoms providing a simplified seed train process.
Figure 4a is a diagram showing scale up issues and factors that negatively affect scale-up optimization related to mixing, shear force, hydro-mechanical stress, aeration, and CO2 stripping, of industrial tall-cylinder stir-tank bioreactors.
Figure 4b is a diagram showing advantages of wide-body bioreactor vessels with inverted frusto-conical bottom for large-scale industrial cell culture.

### DETAILED DESCRIPTION OF THE INVENTION

This invention is based, at least in part, on the discovery that, without using sophisticated control tower and related DO and pH probes, suspension adapted mammalian cells grew well when culture medium surface area/culture medium volume is above 0.14 cm2/cm3. This culture was conducted in a shaker bottle with a bottom air sparging or overlay pure 02 supply at 37°C using a serum-free suspension medium supplemented with 20-25mM HEPES. The above discovery indicated that wide-body culture vessels have advantage for medium surface aeration over traditional tall cylinder stir-tank culture vessels.

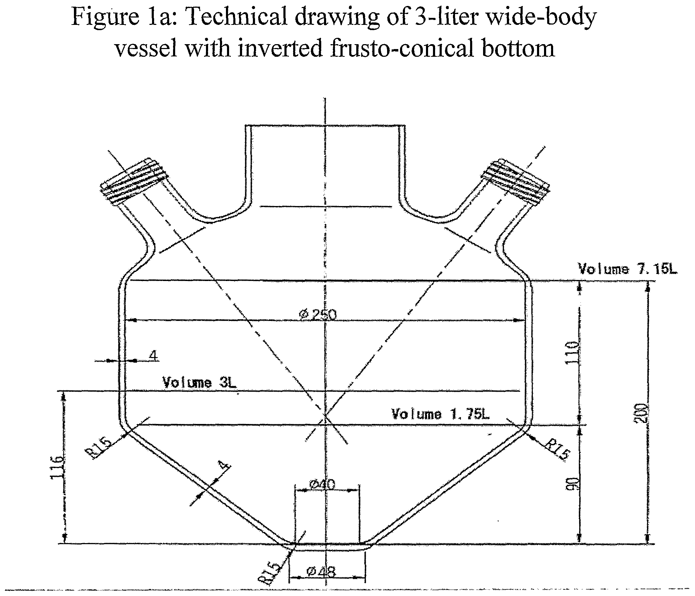

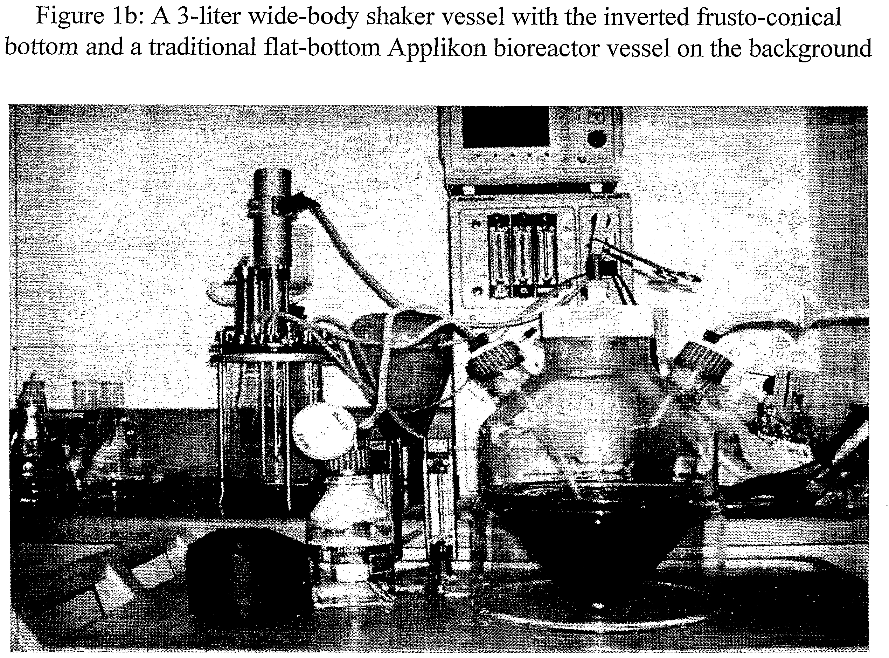

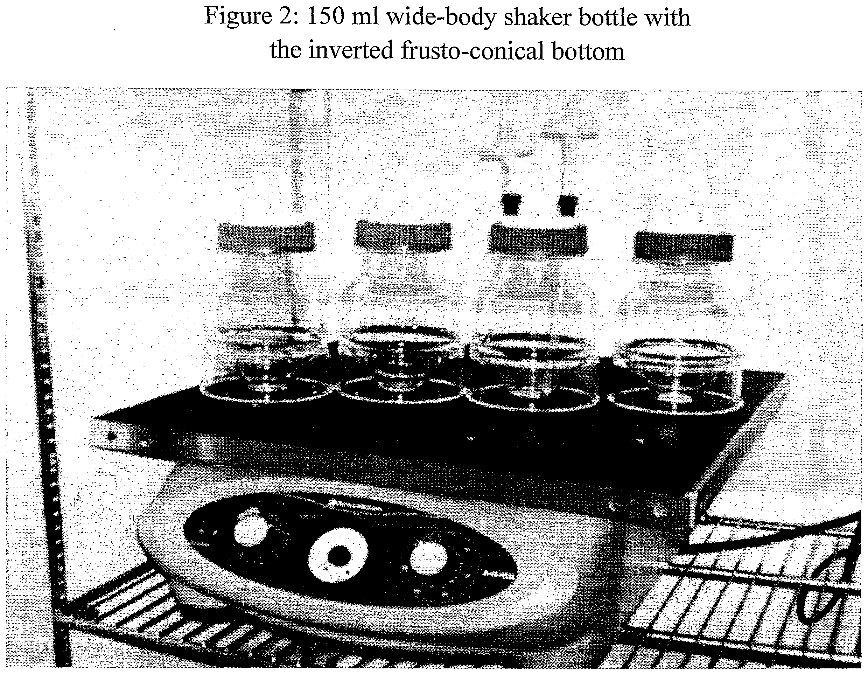

Many robust mammalian cell lines have been developed for serum-free and animal component-free suspension culture. These include CHOD (B11 and DG44), CHOK, and NS0 (serum-free adapted) cells. Also, powerful animal component-free suspension culture media (basal growth and feed) have also been developed. By adding a non-CO2 dependent buffer such as 20 mM HEPES buffer and sufficient surface aeration, sophisticated control tower and related dissolved oxygen (DO) and pH probes become not essential for small volume suspension cultures when wide body culture vessels are employed. The above developments enabled us to freely test and develop simplified and "easy to optimize" bioreactor vessels for serum-free suspension culture of mammalian cells. We have designed and made 3-liter (Figure 1a,b) according to the invention and 150ml (Figure 2) work volume wide-body culture vessels with inverted frusto-conical bottom. These vessels were first used as shaker culture vessels for mammalian cell culture. Surprisingly, they worked much better than conventional Applikon flat-bottom bioreactor vessels of similar volume in term of cell density, recombinant protein productivity, reliability, and user-friendlyness. Clearly, combination of orbital shaking, frusto-conically shaped bottom, wide-body and pH stable culture medium worked in favor of mammalian cell culture and recombinant protein production. The focus was first on a small-scale 150 ml wide-body shaker bottle with an inverted frusto-conical bottom for robustness screening of production cell line candidates. The important invention for this part was the inverted frusto-conical bottom for reduced seed volume for initiation of a cell culture process.

In this study, a wide-body vessel with an inverted frusto-conical bottom (Figure 2) was studied first side by side with a wide-body shaker vessel with a "sharp-point" conical bottom (Coming, cat #431123). Each e vessel has a total volume of 500ml and a designated 150ml work volume. CHO cells expressing recombinant protein VEGFR1-Fc-IL-1ra were used for this study. Fed-batch results in Table 1 indicate that these 150ml shaker vessels were good for small-scale suspension fed-batch study. A small seed volume 7.5-15ml (1/20-1/10 of the 150ml work volume) was achieved for successful initiation of a cell culture process. The shaker vessel with "sharp-point" conical bottom caused cells precipitation particularly cell clusters (Table 1) at the top of the bottom, indicating poor mixing at the region. Thus it was abandoned. Effective cone angles for the culture vessels with conical bottom were also considered. Influence of the cone angle on orbital shaking, culture medium mixing, and seed volume were studied by using plastic conical centrifugation tubes with different cone angles (Table 2). The orbital shaking may not be able to make the culture medium climbing up on the wall easily, if the angle is too narrow (such as <30 degrees) or too wide (such as >70 degrees). According to this invention, the focus was on 3-liter suspension culture vessels with no sophisticated control tower and related DO, pH probes. Aeration was through either air sparging at the lowest point of the inverted frusto-conical bottom for extended air bubble travel time course or pure 02 overlay over large medium surface area.

First, we found that orbital shaking was able to make the culture medium easily climb up onto the wall of the culture vessel, thereby achieving low hydro-mechanical stress and creating a broad thin medium layer for extended surface, greater aeration and better mixing.

In this study, a 3-liter wide-body shaker vessel with an inverted frusto-conical bottom (Figure 1a, b) was constructed and tested with no control tower or related DO probe. Filtered air (containing about 21% of 02) was pumped into this shaker vessel for air sparging at low-flow rate without causing significant cell damage. Alternatively very low flow-rate of pure 02 was used through a tube device at bottom of the vessel (serves as a flow-rate monitor) for generation of larger bubbles or direct 02 overlay.

CHO cells expressing TNFR1-Fc-IL-1ra, IL-18bp-Fc-IL-1ra, VEGFR1-Fc-IL-1ra, and Tie2-Fc-IL-1ra were used for this study. Applikon 2 liter flat-bottom stir-tank bioreactor (Figure 1b background) and 20 liter coy-boy vessel served as flat bottom shaker bottles. Each had a 3 liter work volume culture and was employed as a control.

Table 3, 4, 5 summarized the results from the inverted frusto-conical bottom vessels compared with results from Applikon bioreactor vessels and flat-bottom shaker vessels.

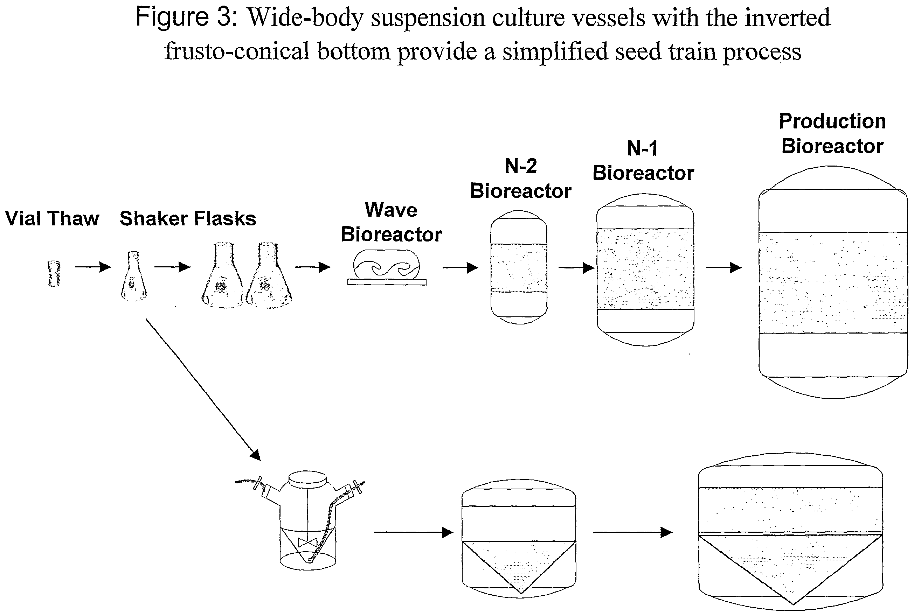

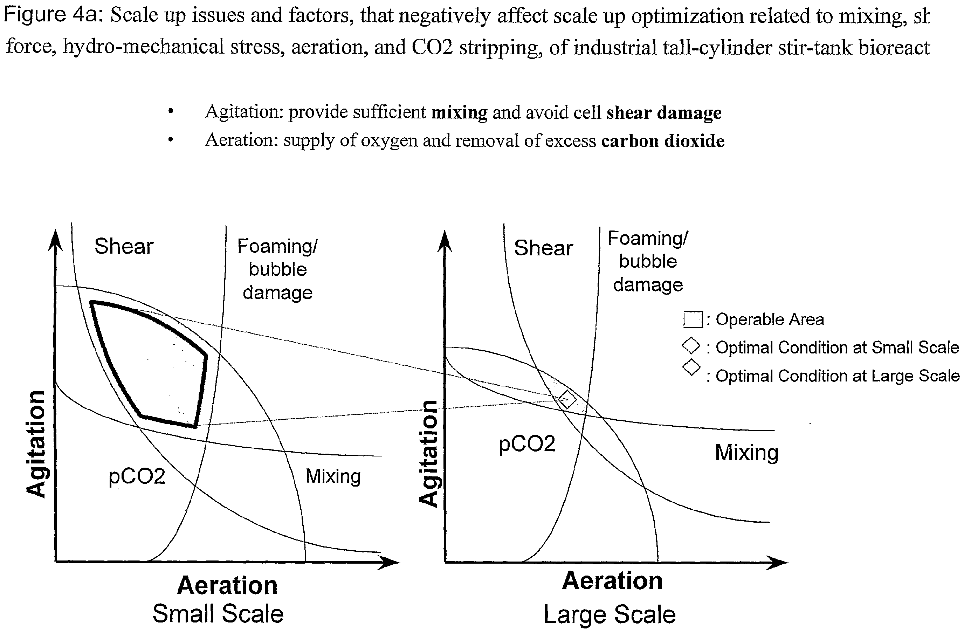

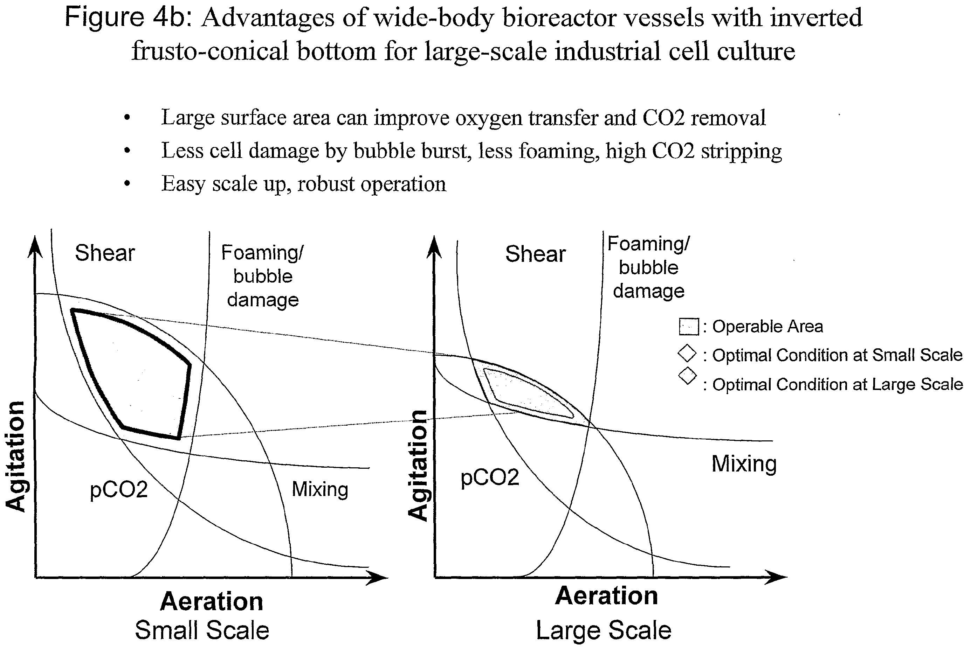

Surprisingly, the wide-body shaker vessel (Table 3) has worked significantly better than flat-bottom Applikon stir-tank (Table 4) and flat-bottom shaker vessels (Table 5). Clearly, orbital shaking motion is able to push culture medium up onto the vessel wall easily with no shear force and less hydro-mechanical stress, and create a broad thin medium layer for greater aeration. Design, analysis and prototype study of a large volume impellor-based industrial stir-tank wide-body bioreactor with an inverted frusto-conical bottom were also considered. Application of the inverted frusto-conical bottom contributes significantly to reduced seed volume (Figure 3) as well as better mixing with less hydro-mechanical stress and possible low shear force. In addition, the larger surface area of the design certainly contributes to better aeration and more effective CO2 stripping (Figure 4a, b). Our goal was to make larger industrial bioreactors stable, easier to optimize and use of significantly reduced seed volume. Figure 4a shows scale up issues and factors that negatively affect scale up optimization related to mixing, shear force, hydro-mechanical stress, aeration, and CO2 stripping. These factors make scale up optimization more difficult particularly for large volume industrial bioreactors. After the analysis, we have concluded that, at least in theory, a wide-body vessel with a conical bottom not only improves scale up optimization (such as better mixing with less hydro-mechanical stress and possible less shear force)(Figure 4b), but also reduces feed volume of impellor driven stir-tank bioreactor vessels (Figure 3). At a given agitation speed, the inverted frusto-conical bottom of the vessel and the increased surface area together improve scale up optimization process.

### Example 1 (comparative)

### Study of 150 ml wide-body shaker vessel with inverted frusto-conical bottom

Use of small-scale shaker vessels for fed-batch culture of CHO production cell line candidates is an important approach to screen production cell lines for robustness and high productivity. In this study, 150 ml wide-body vessels with the inverted frusto-conical bottom (Figure 2) were tested side by side with wide-body shaker bottles with inverted "sharp-point" conical bottom (Corning, Cat #431123). The fed-batch results (Table 1) indicated that these 150ml shaker bottles were suitable for small-scale suspension fed-batch study. Small seed volume (1/20 of the work volume) was achieved. Expression titers were comparable to the 2-liter bioreactor. The shaker vessel with the inverted "sharp-point" conical bottom caused obvious cells precipitation particularly large size cell clusters showing poor mixing in the bottom tip area. For this reason, we abandoned use of the inverted "sharp-point" conical bottom and adapted the inverted frusto-conical bottom.

**Table 1: The culture vessels were placed on a shaker platform at speed 100 rpm. Feed (4.5ml) started at day 7 when batch culture volume reached 90ml for two days.**

| **Vessel bottom** | **Cell line** | **Seed volume** | **Seed density** | **Before feed at Day 7** | **After feed at day 9** | **End of culture at day 11** | **Expression titer (mg/L)** | **Cells or cell cluster precipitation at bottom** |
|---|---|---|---|---|---|---|---|---|
| Frusto-conical bottom | CHO expressing VEGFR1-Fc-IL-1ra | 15ml | 1±0.2x10-6 cells/ml | 6.0±0.8x10-6 cells/ml | 10.2±1.5x10-6 cells/ml | 7.1±0.9x10-6 cells/ml | 128±8.0 | No |
| Sharp-point conical bottom | CHO expressing VEGFR1-Fc-IL-1ra | 15ml | 1±0.2x10-6 cells/ml | 6.5±0.7x10-6 cells/ml | 9.9±1.3x10-6 cells/ml | 6.8x10-6 cells/ml | 118±0.6 | Yes |

### Example 2 (comparative)

### Study of culture vessels with the inverted conical bottom by different cone angles

Influence of cone angle of the conical bottom on orbital shaking, medium mixing, and seed volume were studied by using plastic centrifugation tubes with different cone angles on shaker platform (Table 2). The orbital shaking might not be able to make the culture volume climbing up on vessel wall easily and forming a broad thin layer with extended medium surface area, if the angle was too narrow or too wide. For example, it was not significantly different from the flat bottom vessels if the angle was too wide (such as >70 degrees). It was also not much different from flat bottom vessels if the angle was too narrow (such as <30 degrees). Results in Table 3 suggested that 30 degree of the angle from the cylinder wall perhaps was probably the minimum angle for effective orbital shaking and mixing. According to calculation, > 70 degrees of cone angle, there was no significant reduced seed volume was achieved from that of regular round bottom culture vessels, thus being abandoned.

**Table 2: Study of vessels with the conical bottom by different angles on effective orbital shaking and mixing, and seed volume. Note: CHO cells expressing TNFR2-Fc-IL-1ra were used for this study.**

| | | | | | | |
|---|---|---|---|---|---|---|
| Brand and Cat# | Measured angle | Cone angle description | Total volume | Effect on orbital shaking | Effect on mixing | Effect on seed volume |
| Corning Cat#43776 | Conical bottom is 30 degree angle from the cylinder wall | Narrow | 250 ml | Not shaking well | Precipitation at the apex | No problem |
| Kendall Cat#20820 | Frusto-conical bottom is 40 degree angle from the cylinder wall | Wide | 50 ml | Shaking well | No precipitation at both low and high speed | No problem |
| Coming Cat# 431123 | Conical bottom is 40 degree angle from the cylinder wall | Wide | 500 ml | Shaking great | Precipitation at the apex at low speed | No problem |
| Custom-made | Frusto-conical bottom is 40 degree angle from the cylinder wall | Wide | 500 ml | Shaking great | No precipitation at both low and high speed | No problem |
| Custom-made | Frusto-conical bottom is 45 degree angle from the cylinder wall | Wide | 3000ml | Shaking great | No precipitation at both low and high speed | No problem |

### Example 3 (invention)

### Study of 3-liter wide-body shaker vessel with inverted frusto-conical bottom

3-liter wide-body shaker vessel with inverted frusto-conical bottom together with 02 tube bubbling device or air sparging was assembled (Figure 1a, b). The culture medium at work volume has larger surface area for medium 02 uptake (>0.143cm2 surface area per cm3 medium volume). The inverted frusto-conical bottom makes a low-positioned air sparging device possible for extended air (containing about 21% of 02) traveling time course (Figure a, b). The 02 bubble tube device at the bottom serves as a low flow meter monitor for very low flow rate of pure 02 medium surface supply. The orbital shaking motion is able to make the culture volume climbing up on the conical bottom wall easily and creating a broad thin medium layer for extended medium surface area and better aeration.

We have been routinely using CHO cell lines expressing TNFR1-Fc-IL-1ra, IL-18bp-Fe-IL-1ra, VEGFR1-Fc-IL-1ra, and Tie2-Fc-IL-1ra for production of animal testing materials. The above 3-liter wide-body shaker vessel with inverted frusto-conical bottom, Applikon 2 liter flat-bottom stir-tank bioreactor and 20 liter shaker-based coy-boy culture vessel were employed for the routine production. Table 3, 4, 5 summarized all the results from the inverted frusto-conical bottom vessels comparing with results from flat-bottom Applikon bioreactor vessel and flat-bottom shaker vessel.

Surprisingly, the wide-body shaker vessel with inverted frusto-conical bottom (Table 3) worked significantly better than flat-bottom Applikon stir-tank (Table 4) and shaker vessel (Table 5). Clearly, orbital shaking motion is able to push culture medium climbing up on the vessel wall easily with no shear force and low hydro-mechanical stress, and creating a broad thin medium layer for greater aeration.

The wide-body shaker culture vessel with inverted frusto-conical bottom was designed to be better than recently developed "Wave" plastic bag bioreactor. Our analysis showed that it is similar to "Wave" with large surface area for 02 uptake and CO2 remival. However, the orbital shaking together with frusto-conical bottom of the vessel makes the culture medium climbing up on the vessel wall easily and creating a broad thin layer of culture medium for extended medium surface aeration. Similar to"Wave", it is "single use" plastic vessels. However, it is better than "Wave" due to significantly reduced seed volume for initiating a given cell culture process. It is also better than "Wave" that an air sparging device is placed at the frusto-conical bottom for significantly extend air bubble travel time course. Although we did not compare "Wave" directly with the wide-body culture vessel with inverted frusto-conical bottom in terms of cell density, cost-effectiveness and user-friendlyness, all the indirect data pointed that the wide-body culture vessel with inverted frusto-conical bottom is much better than "Wave" in all the aspects mentioned.

**Table 3: 3-liter wide-body shaker vessel with inverted frusto-conical bottom together with 02 tube bubbling device or air sparging was used for routine fed-batch production of animal testing materials by using CHO cell lines expressing TNFR1-Fc-IL-1ra, IL-18bp-Fc-IL-1ra, VEGFR1-Fc-IL-1ra, and Tie2-Fc-IL-1ra.**

| Gas supply mode | CHO cells expressing | Seed volume | The highest cell density | Total culture length | Packed cell volume (pcv) % | Final dot blot titer (+ after dilution) | Final purification yield |
|---|---|---|---|---|---|---|---|
| Low-positioned air sparging | TNFR1-Fc-IL-1ra | 300ml | 20.4±6.0x10-6 cells/ml | 13 days | 4.2% | 518 mg/ml | 77mg/L |
| Low-positioned air sparging | IL-18bp-Fc-IL-1ra | 300ml | 18.8±0.6x10-6 cells/ml | 14 days | 4.0% | 518 mg/ml | 65 mg/L |
| Low-positioned air sparging | VEFR1-Fc-IL-1ra | 300ml | 15.0±0.3x10-6 cells/ml | 14 days | 3.5% | 256 mg/ml | 66 mg/L |
| Low-positioned air sparging | Tie2-Fc-IL-1ra | 300ml | 10.0±0.3x10-6 cells/ml | 15 | 2.2% | 128 mg/ml | 42 mg/L |
| O2 Overlay | TNFR1-Fc-IL-1ra | 300ml l | 15.0±0.3x10-6 cells/ml | 14 days | 3.4% | 518 mg/ml | 73 mg/L |
| O2 Overlay | IL-18bp-Fc-IL-1ra | 300ml | 9.0±0.9x10-6 cells/ml | 15 days | 2.3% | 518 mg/ml | 66 mg/L |
| O2 Overlay | VEFR1-Fc-IL-1ra | 300ml | 18.0±0.7x10-6 cells/ml | 13 days | 3.8% | 256 mg/ml | 67 mg/L |
| O2 Overlay | Tie2-Fc-IL-1ra | 300ml | 12.0±0.5x10-6 cells/ml | 14 days | 2.8% | 128 mg/ml | 39 mg/L |

**Table 4: Applikon 2 liter flat-bottom stir-tank bioreactor vessel was routinely employed for routine Fed-batch production of animal testing materials by using CHO cell lines expressing TNFR1-Fc-IL-1ra, IL-18bp-Fc-IL-1ra, VEGFR1-Fc-IL-1ra, and Tie2-Fc-IL-1ra. Some of the results summarized here as controls.**

| Gas supply mode | CHO cells expressing | Seed volume | The highest cell density | Total culture length | Packed cell volume (pcv) % | Final dot blot titer (+ after dilution) | Final purification yield |
|---|---|---|---|---|---|---|---|
| Low-positioned air sparging | TNFR1-Fc-IL-1ra | 800ml | 9.4±6.0x10-6 cells/ml | 13 days | 2.2% | 256 mg/ml | 44 mg/L |
| Low-positioned air sparging | IL-18bp-Fc-IL-1ra | 800ml | 8.8±0.7x10-6 cells/ml | 12 days | 2.0% | 256 mg/ml | 41 mg/L |
| Low-positioned air sparging | VEFR1-Fc-IL-1ra | 800ml | 10.0±0.7x10-6 cells/ml | 12 days | 2.3 | 256 mg/L | 42 mg/L |
| Low-positioned air sparging | Tie2-Fc-IL-1ra | 800ml | N/A | N/A | Did not grow | N/A | N/A |
| Low-positioned pure O2 sparging | TNFR1-Fc-IL-1ra | 800ml | 8.0±0.3x10-6 cells/ml | 13 days | 1.8% | 256 mg/ml | 46 mg/L |
| Low-positioned pure O2 sparging | IL-18bp-Fc-IL-1ra | 800ml | 9.0±0.9x10-6 cells/ml | 12 days | 2.0% | 256 mg/ml | 42 mg/L |
| Low-positioned pure O2 sparging | VEFR1-Fc-IL-1ra | 800ml | N/A | N/A | contaminated | N/A | N/A |
| Low-positioned pure O2 sparging | Tie2-Fc-IL-1ra | | 9.1±0.310-6 cells/ml | 13 days | 2.2% | 128 mg/L | 27 mg/L |

**Table 5: 20 liter shaker-based coy-boy culture vessel with 02 tube bubbling device or air sparging was also routinely employed for routine Fed-batch production of animal testing materials by using CHO cell lines expressing TNFR1-Fc-IL-1ra, IL-18bp-Fc-IL-1ra, VEGFR1-Fc-IL-1ra, and Tie2-Fc-IL-1ra. Some of the results summarized here as controls.**

| Gas supply mode | CHO cells expressing | Seed volume | The highest cell density | Total culture length | Packed cell volume (pcv) % | Final dot blot titer (+ after dilution) | Final purification yield |
|---|---|---|---|---|---|---|---|
| Low-positioned air sparging | TNFR1-Fc-IL-1ra | 800ml | 9.0±3.0x10-6 cells/ml | 9 days | 2.0% | 256 mg/ml | 40 mg/L |
| Low-positioned air sparging | IL-18bp-Fc-IL-1ra | 800ml | 8.8±0.6x10-6 cells/ml | 8 days | 2.2% | 256 mg/ml | 42 mg/L |
| Low-positioned air sparging | VEFR1-Fc-IL-1ra | 800ml | 10.0±0.3x10-6 cells/ml | 8 days | 2.2% | 256 mg/ml | 38 mg/L |
| Low-positioned air sparging | Tie2-Fc-IL-1ra | 800ml | 9.0±0.3x10-6 cells/ml | 9 days | 2.0% | 128 mg/ml | 26 mg/L |
| O2 overlay | TNFR1-Fc-IL-1ra | 800 ml | 10.0±0.3x10-6 cells/ml | 9 days | 2.4% | 256 mg/ml | 41 mg/L |
| O2 overlay | IL-18bp-Fc-IL-1ra | 800ml | 9.0±0.9x10-6 cells/ml | 9 days | 2.3% | 256 mg/ml | 39 mg/l |
| O2 overlay | VEFR1-Fc-IL-1ra | 800ml | 8.9±0.7x10-6 cells/ml | 10 days | 2.2% | 256 mg/ml | 40 mg/L |
| O2 overlay | Tie2-Fc-IL-1ra | 800ml | 10.0±0.5x10-6 cells/ml | 10 days | 2.2% | 64 mg/ml | 27 mg/L |

### Example 4

Two detailed examples fed-batch runs of TNFR1-Fc-IL-1ra by using 3-liter wide-body shaker vessel with inverted frusto-conical bottom and 2-liter Applikon flat-bottom bioreactor
3-liter wide-body shaker vessel with inverted frusto-conical bottom together with 02 tube bubbling device, and 2-liter flat-bottom Applikon bioreactor was used to produce TNFR1-Fc-IL-1ra in serum-suspension medium. Fed-batch mode was used. Table 6 shows the results. Note: 2% pcv = 9.6x10-6 cells/ml. Clear advantage of 3-liter wide-body shaker vessel with inverted frusto-conical bottom was demonstrated over 2-liter Applikon flat-bottom bioreactor.

Table 6: 3-liter wide-body shaker vessel with inverted frusto-conical bottom together with 02 tube bubbling device, and 2-liter flat-bottom Applikon bioreactor was used to produce TNFR1-Fc-IL-1ra in serum-suspension medium. Fed-batch mode was used.

**Table 6 shows the results. Note: 2% pcv (packed cell volume) = 9.6x10-6 cells/ml. Clear advantage of 3-liter wide-body shaker vessel with inverted frusto-conical bottom was demonstrated over 2-liter Applikon flat-bottom bioreactor.**

| Day | 1 | 2 | 3 | 4 | 5 | 6 | 7 | 8 | 9 | 10 | 11 | 12 |
|---|---|---|---|---|---|---|---|---|---|---|---|---|
| frusto-conical bottom | 0.2% pcv seeded | 0.2% pcv | 0.5% pcv | 1.0% pcv | 1.8% pcv | 2.2% pcv | 2.2% pcv | 2.3% pcv | 2.8% pcv | 3.0% pcv | 2.6% pcv | 2.2% pcv |
| Applikon flat-bottom | 0.2%pcv seeded | 0.2% pcv | 0.4% pcv | 0.9% pcv | 1.6% pcv | 2.0% pcv | 2.1% pcv | 2.0% pcv | 1.8% pcv | 1.6% pcv | | |

## Claims

1. A cell culture vessel comprising a housing defining a chamber that includes an inverted frusto-conical bottom and a cylindrical body that extends upward from the inverted frusto-conical bottom, the chamber having a vertical axis, wherein the inverted frusto-conical bottom has a slope, a top portion and an opposite bottom portion, said bottom portion having a larger inner-diameter than the top portion, and wherein the dimensions of the inner-diameter of the top portion, of the inner-diameter of the bottom portion, of the inner-diameter of the cylindrical body, of the height of the inverted frusto-conical bottom and of the height of the cylindrical body are 40 mm, 250 mm, 250 mm, 90 mm and 110 mm, respectively.

2. A system for culturing cells, comprising a vessel of claim 1 and an orbital shaking platform or impellers.

3. A method of culturing living cells, comprising the steps of:
(a) providing a vessel of claim 1;
(b) introducing a work liquid culture medium to the vessel;
(c) introducing to the vessel a seeding culture containing cells of interest; and
(d) culturing the cells under a suitable condition, wherein the O₂ breathing area/culture medium volume ratio is no less than 0.14 cm²/cm³.

4. The method of claim 3, wherein the ratio of work liquid culture medium volume/seeding culture volume is no greater than 1/10.

5. The method of claim 3, wherein the work liquid culture medium contains a serum-free medium having CO₂-non-dependent 20 mM HEPES or equivalents to keep culture pH stable.

## Patentansprüche

1. Zellkulturgefäß mit einem Gehäuse, das eine Kammer definiert, die einen umgekehrten kegelstumpfförmigen Boden und einen zylindrischen Körper aufweist, der sich von dem umgekehrten kegelstumpfförmigen Boden nach oben erstreckt, wobei die Kammer eine vertikale Achse aufweist, wobei der umgekehrte kegelstumpfförmige Boden eine Schräge, einen oberen Abschnitt und einen gegenüberliegenden Bodenabschnitt aufweist, wobei der Bodenabschnitt einen größeren Innendurchmesser als der obere Abschnitt aufweist und wobei die Abmessungen des Innendurchmessers des oberen Abschnitts, des Innendurchmessers des Bodenabschnitts, des Innendurchmessers des zylindrischen Körpers, der Höhe des umgekehrten kegelstumpfförmigen Bodens und der Höhe des zylindrischen Körpers jeweils 40 mm, 250 mm, 250 mm, 90 mm bzw. 110 mm sind.

2. System zur Kultivierung von Zellen, umfassend ein Gefäß nach Anspruch 1; eine orbital schüttelnde Plattform; oder Impelloren.

3. Verfahren zur Kultivierung lebender Zellen, umfassend die Schritte:
(a) Bereitstellen eines Gefäßes nach Anspruch 1;
(b) Einführen eines Arbeitsflüssigkeitskulturmediums in das Gefäß;
(c) Einführen einer Saatkultur, die Zellen von Interesse enthält, in das Gefäß; und
(d) Kultivieren der Zellen unter einer geeigneten Bedingung, wobei das Verhältnis von O₂-Atmungsfläche/Kulturmediumvolumen nicht weniger als 0,14 cm²/cm³ beträgt.

4. Verfahren nach Anspruch 3, **dadurch gekennzeichnet, dass** das Verhältnis von Arbeitsflüssigkeitskulturmediumvolumen/Saatkulturvolumen nicht größer als 1/10 ist.

5. Verfahren nach Anspruch 3, **dadurch gekennzeichnet, dass** das Arbeitsflüssigkeitskulturmedium ein serumfreies Medium mit CO₂-nicht-abhängigen 20 mM HEPES oder Äquivalenten enthält, um den pH-Wert der Kultur stabil zu halten.

## Revendications

1. Un récipient de culture cellulaire comprenant une envelope définissant une chambre qui comprend un fond tronconique inversé et un corps cylindrique qui s'étend vers le haut à partir du fond tronconique inversé, la chambre ayant un axe vertical, dans lequel le fond tronconique inversé a une pente, une partie supérieure et une partie inférieure opposée, ladite partie inférieure ayant un diamètre intérieur plus grand que la partie supérieure, et dans lequel les dimensions du diamètre intérieur de la partie supérieure, du diamètre intérieur de la partie inférieure, du diamètre intérieur du corps cylindrique, de la hauteur du fond tronconique inversé et de la hauteur du corps cylindrique sont respectivement de 40 mm, 250 mm, 250 mm, 90 mm et 110 mm.

2. Système pour cultiver des cellules, comprenant un récipient selon la revendication 1; une plate-forme de secousse orbitale; ou des hélices.

3. Procédé de culture de cellules vivantes, comprenant les étapes de:
(a) fournir un récipient de la revendication 1;
(b) introduire un milieu liquide de culture de travail dans le récipient;
(c) introduire dans le récipient une culture d'ensemencement contenant des cellules d'intérêt; et
(d) cultiver des cellules dans un condition approprié, dans lequel le rapport de surface de respiration d'O₂/ volume de milieu de culture n'est pas inférieur à 0,14 cm²/cm³.

4. Procédé selon la revendication 3, dans lequel le rapport du volume de milieu liquide de culture de travail/ volume de culture d'ensemencement n'est pas supérieur à 1/10.

5. Procédé selon la revendication 3, dans lequel le milieu liquide de culture de travail contient un milieu sans sérum ayant des HEPES 20 mM non dépendants de CO₂ ou des équivalents pour maintenir le pH de la culture stable.
